# DEMANDE DE BREVET EUROPEEN

(11) **EP 1 997 516 A1**
(43) Date de publication de la demande: **03.12.2008**
(21) Numéro de dépôt: 08156732.3
(22) Date de dépôt: 22.05.2008
(51) Int. Cl.: A61L 2/07, A61L 2/26

(54) **Procédé et dispositif de réduction de l'eau liquide dans les containers lors de la stérilisation d'équipements à la vapeur d'eau**

(30) Priorité: 24.05.2007 FR 0703694
(71) Demandeur: Sterlab, 06220 Vallauris (FR)
(72) Inventeur: Martel, Paul, 83700 Saint Raphael (FR)
(74) Mandataire: Somnier, Jean-Louis

(57) **Abrégé**

Dispositif absorbant d'eau pour container (5) de stérilisation d'équipements, destiné pour cela à être placé dans un appareil assurant la stérilisation par la vapeur d'eau, tel qu'il a des capacités d'absorption de l'eau par capillarité le dispositif étant de forme amincie entre deux faces principales (2₁, 2₂) au moins quasi-parallèles, et disposées en position verticale.

## Description

La présente invention a pour objet un procédé et un dispositif de réduction de l'eau liquide dans les containers lors de la stérilisation d'équipements à la vapeur d'eau.

Le secteur technique de l'invention est le domaine de la stérilisation d'équipements tel qu'en particulier des ustensiles médicaux et d'hôpitaux, effectuée dans des appareils fermés tels que des autoclaves de type à prévide et utilisant la vapeur d'eau saturée sèche comme un gaz stérilisant, l'énergie de stérilisation venant principalement de la chaleur libérée par la vapeur lors de sa condensation sur les ustensiles ou instruments.

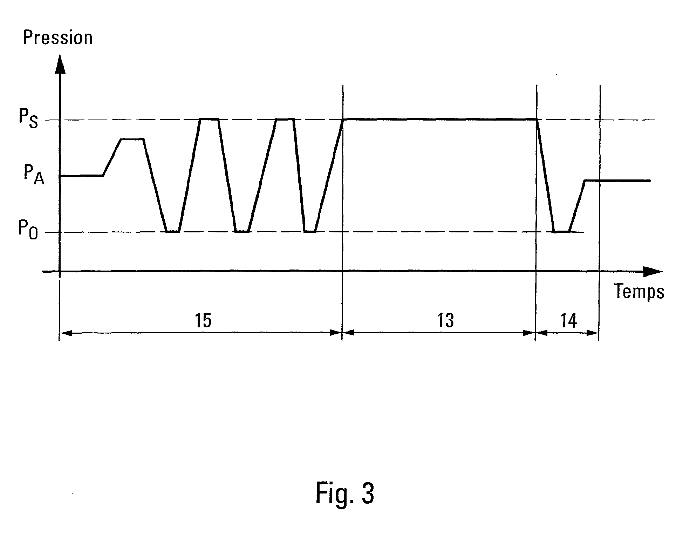

Il est connu que de tels appareils autoclaves fonctionnent, à titre d'exemple, suivant des cycles de pression et de température tels que représentés sur la figure 3 : une première phase dite de prétraitement constituée alternativement et successivement d'injections de vapeur et de mises sous vide permet de mettre à température l'ensemble des ustensiles et des containers qui les contiennent et de revaporiser et évacuer l'eau qui a pu se condenser à partir de la vapeur pendant cette phase; ainsi lors de la phase suivante de stérilisation proprement dite, à la pression et à la température de la vapeur saturée, soit par exemple comme cela est souvent utilisé en France, à deux bars relatifs et 134°C (il pourrait être également utilisé un bar relatif et 121°C, la pression de stérilisation étant directement liée à la température) ladite vapeur assure pleinement sa fonction de gaz stérilisant mais à condition qu'il n'y ait pas d'excès d'eau sous forme liquide en contact avec les instruments, celle-ci ayant été pratiquement éliminée lors de la phase de prétraitement, la quantité d'eau qui se condense sur les instruments et qui ne se revaporiserait pas lors de la phase de stérilisation étant théoriquement faible.

Cependant, la plupart des fabricants et des utilisateurs cherchent à diminuer la durée de cette phase de prétraitement pour une meilleure optimisation de l'utilisation des autoclaves : aussi, suivant le type et la quantité d'équipements à stériliser disposés dans le container, il peut encore rester une certaine quantité d'eau issue de la condensation dans le fond de celui-ci et même en suspension dans la vapeur sous forme de gouttelettes (voir ci-après) au début de la phase de stérilisation.

De même, les utilisateurs, personnels hospitaliers par exemple, peuvent charger excessivement les containers et rendre difficile voire impossible pendant le prétraitement la mise en température d'une charge rendue ainsi trop massive. La phase de stérilisation générera dans ce cas de l'eau issue de la condensation qui ne pourra s'évacuer et se déposera la encore au fond du container.

Pour éviter en ces cas une trop grande accumulation d'eau qui pourrait nuire à une bonne stérilisation, certaines solutions ont été proposées telles que :
- la réalisation de containers à double fond qui reçoivent cette eau et la stockent hors du contact de la charge pendant la stérilisation.
- la réalisation de containers avec systèmes de soupape pour évacuer l'eau mais de tels containers assez complexes n'ont pas eu de succès commercial.
- le rajout d'un tapis de type éponge déposé sous les équipements à stériliser dans le fond des containers, et apte à s'imbiber de l'eau pouvant s'y accumuler, mais une telle solution ne permet pas de réduire une grande quantité d'eau, ce qui peut être insuffisant suivant le cycle de stérilisation et la quantité d'équipements concernés.

D'autre part, la vapeur présente dans le container peut être elle-même surchargée en gouttelettes d'eau liquide issues soit du fond du container où un excès d'eau s'est déposé comme cela est indiqué plus haut soit de la condensation résiduelle présente sur les instruments placés dans le container soit du circuit de génération de vapeur lui-même si les séparateurs de phase liquide/vapeur ne sont pas suffisamment efficace ou si les canalisations sont trop longues et permettent la recondensation dans les circuits d'alimentation en vapeur.

Dans tous les cas, cette vapeur n'est plus dite saturée sèche mais saturée humide ou sursaturée et ses propriétés thermodynamiques lui confèrent un pouvoir stérilisant inférieur par rapport à la vapeur saturée sèche. Le titre de vapeur définit le pourcentage d'eau liquide présente dans la vapeur saturée. La vapeur saturée sèche ne contient pas d'eau liquide : son titre vaut 1. Une vapeur de titre 0,98 contient 2 % d'eau liquide. Selon la norme EN 285 : « Stérilisation à la vapeur d'eau, Grands stérilisateurs », la vapeur utilisée pour la stérilisation doit avoir un titre supérieur ou égal à 0,95.

Le problème posé est donc d'empêcher lors de la phase de stérilisation une trop grande présence d'eau liquide soit dans le fond des containers soit dans la vapeur présente dans le container pour permettre une bonne efficacité de la stérilisation, c'est-à-dire en évitant que les équipements à stériliser soient en contact avec cette eau de façon excessive, et cela sans modification des containers existants, sans augmenter la durée de traitement préalable et même en la diminuant au maximum, et avec des moyens simples, faciles à mettre en oeuvre dans tout container.

Une solution au problème posé est un procédé de réduction de la quantité d'eau s'accumulant dans le fond des containers de stérilisation d'équipements et dans la vapeur servant à la stérilisation de ceux-ci, lesquels containers étant destinés pour cela à être placés dans des appareils assurant la stérilisation par la vapeur d'eau, tel que, suivant l'invention :
- on glisse verticalement, à l'intérieur et préalablement à la fermeture du container, entre la paroi de celui-ci et les équipements à stériliser, au moins un dispositif absorbant d'eau apte à absorber l'eau par capillarité depuis au moins sa partie inférieure,
- on positionne ladite partie inférieure près du fond du container, et au mieux au plus près de ce fond.

Suivant un mode particulier de réalisation, le dispositif est également apte à absorber l'eau par capillarité par au moins une des ses faces latérales.

Pour les containers comprenant au moins un panier intérieur recevant les équipements à stériliser, on glisse verticalement entre la paroi du container et celle du panier intérieur au moins un dispositif tel que défini ci-dessus.

Un tel dispositif suivant l'invention a des capacités d'absorption de l'eau par capillarité depuis au moins sa partie dite inférieure quand celle-ci est positionnée pour cela vers le bas, le dispositif étant de forme dite amincie entre deux faces principales au moins quasi-parallèles, et qui sont alors disposées en position verticale ; et l'une au moins de ces faces latérales peut être, au moins sur une partie de sa surface, perméable à l'eau.

Dans un mode préférentiel de l'invention on glisse plusieurs dispositifs dans le container de telle sorte que la capacité totale d'absorption d'eau par ces dispositifs soit supérieure à la quantité totale d'eau pouvant s'accumuler dans le container pendant un cycle de stérilisation et qui n'a pas été évacuée pendant la phase de traitement ou qui reste présente dans la vapeur.

Dans le mode de réalisation préférentiel décrit ci-dessus, dans lequel le dispositif absorbant comporte deux faces principales au moins quasi-parallèles, l'épaisseur qui les sépare est au plus égale au cinquième (1/5) et même au dixième (1/10) de la plus grande dimension de ces faces principales, permettant de qualifier un tel dispositif de forme « amincie », et l'une de ces faces principales est disposée en regard de la paroi du container. Dans le cas où le container comporte au moins un panier intérieur recevant les équipements à stériliser, on dispose les deux dites faces principales du dispositif face aux parois du container et du panier.

Le résultat est un nouveau procédé et dispositif de réduction de la quantité d'eau liquide présente dans les containers lors de la stérilisation d'équipements à la vapeur d'eau, qui répond au problème posé d'une façon simple et efficace.

La description et les dessins suivants représentent un exemple de la réalisation de l'invention mais n'ont aucun caractère limitatif : d'autres réalisations sont possibles dans le cadre de la portée et de l'étendue de cette invention, en particulier en choisissant d'autres matériaux que ceux décrits dans cet exemple de la réalisation, tel que par exemple de la céramique ayant les mêmes capacités d'absorption d'eau par capillarité que dans le mode de réalisation décrit, et ne nécessitant pas de fourreau de protection et de maintien ou de filtre. De même, la forme générale représentée parallélépipédique et dite « amincie » pourrait être différente.
La figure 1 est une vue en coupe d'un exemple de container de stérilisation dans lequel est glissé au moins un dispositif suivant l'invention en coupe suivant le plan I, I' de la figure 2.
La figure 2 est une vue perspective simplifiée d'un dispositif suivant l'invention.
La figure 3 est une représentation graphique d'un exemple le processus de fonctionnement d'un autoclave de stérilisation.

Dans un tel mode de fonctionnement comme déjà indiqué précédemment une première phase 15 de prétraitement comporte plusieurs cycles de variation de pression (en ordonnée) entre celle de vapeur saturée Pₛ a la température de stérilisation choisie tel que à 134°C comme cité précédemment et, soit le vide P₀ soit la pression atmosphérique Pₐ pour revaporiser et évacuer l'eau qui est générée pendant cette phase de prétraitement du fait de la condensation de la vapeur en particulier sur les parois du container et des ustensiles tant que ceux-ci sont plus froids que la température de cette vapeur saturée.

A la fin de cette phase de prétraitement 15 (l'axe des abscisses étant celui du temps), il est alors maintenu, pendant la phase de stérilisation proprement dite 13, la température de vapeur saturée à la pression correspondante, soit à titre d'exemple pour 134°C à deux bars relatifs comme indiqué précédemment, pendant une durée d'au moins dix-huit minutes pour satisfaire aux conditions de stérilisation de la réglementation française, mais ces conditions peuvent varier selon les pays et la température retenue, avant de passer à une phase 14 dite de séchage.

Comme indiqué précédemment, l'objectif est de diminuer le temps total de ce processus de stérilisation sur lequel on ne peut en fait agir qu'en diminuant la phase 15 de traitement au risque de ne pas avoir éliminé à la fin de cette phase toute l'eau présente dans le container 5.

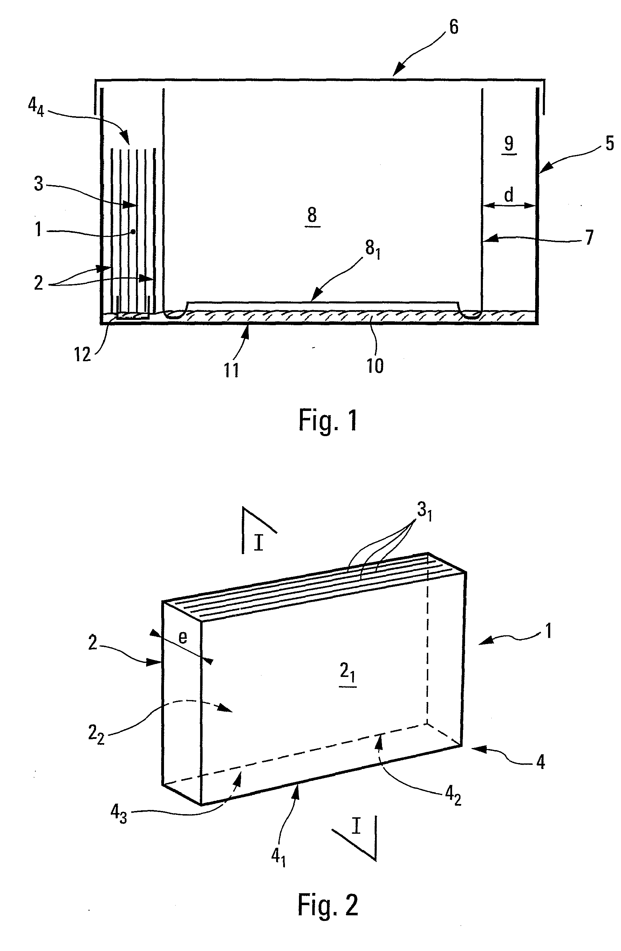

Pour réduire alors cette quantité d'eau qui peut rester encore à la fin de cette phase 15 et celle qui peut continuer à s'accumuler lors de la phase 13 en particulier si la vapeur contient des goutellettes, on glisse suivant l'invention et tel que représenté sur la figure 1, verticalement à l'intérieur et préalablement à la fermeture du container 5 entre la paroi de celle-ci et les équipements à stériliser, qui peuvent être déposés au moins dans un panier intérieur 7, ou plusieurs superposés, au moins un dispositif 1 absorbant d'eau, et en ce cas de dimensions compatibles avec l'espace 9 disponible entre la paroi du container et celle du panier intérieur pour pouvoir y être glissé.

Un tel dispositif, tel que représenté sur la figure 2 et en position d'utilisation suivant la figure 1, est apte à absorber l'eau par capillarité depuis au moins sa partie inférieure 4 positionnée près du fond 11 du container 5 et/ou depuis ses faces latérales: la face inférieure 4₃ de cette partie inférieure 4 étant disposée de préférence à une hauteur inférieure à la distance séparant le fond 8₁ du panier 8 et ledit fond 11 du container 5, afin que l'eau 10 pouvant s'accumuler contre ce dit fond ne puisse pas atteindre le fond 8₁ du panier 8 ; on évite ainsi que les ustensiles placés dans ce panier puissent être mouillés pendant la phase de stérilisation, assurant une meilleure efficacité de celle-ci.

De plus, le dispositif selon l'invention permet de revaporiser l'eau par sa partie supérieure 4₄ pendant la phase de séchage 14, permettant au dispositif de continuer à absorber de l'eau par sa partie inférieure 4 et/ou par ses faces latérales, favorisant l'élimination de l'eau résiduelle et améliorant ainsi le séchage : cette revaporisation peut être aidée en rajoutant au moins une surface métallique disposée parallèlement et entre les faces principales 2₁, 2₂ ; cette surface métallique qui peut être composée d'une feuille telle qu'en aluminium, glissée au milieu du matériau absorbant, fait en effet circuler la chaleur et la transmet à celui-ci du fait de sa bonne conductivité et capacité calorifique : cet apport de chaleur facilite alors la revaporisation de l'eau absorbée par le dispositif selon l'invention.

Le dispositif absorbant 1 tel que représenté sur la figure 1, et apte à être utilisé suivant le procédé tel que décrit précédemment, est semi-rigide pour pouvoir être plus facilement manipulable et il est apte à absorber en eau au moins 50% de son poids sec et même de préférence au moins la totalité de son poids sec ; cette absorption se réalise par capillarité de préférence en raison de moins d'une demi-heure pour 5 cm de hauteur de montée d'eau dans le dispositif, et de préférence 10 cm.

Suivant les modes de représentation des figures 1 et 2, le dispositif forme un bloc de forme générale parallélépipédique, composé d'au mois de trois matériaux dont l'un extérieur 2 enveloppe l'autre intérieur 3 en laissant au moins une zone perméable à l'eau, dans la partie dite inférieure 4 du dispositif et/ou dans ses faces latérales: cette zone peut être située sur la face 4₃ inférieure du dispositif mais peut être également sur les faces latérales telles que les faces principales 2₁, 2₂ et sur des parties de surface contigües à leur bord inférieur 4₁, 4₂... .

Le matériau 2 extérieur pouvant être perméable à l'eau est au moins étanche aux particules du matériau interne 3 empêchant ainsi les particules dudit matériau délitées par l'eau absorbée de se répandre dans le container ; ce matériau 2 forme un fourreau de protection et de maintien et peut donc permettre depuis toute sa surface extérieure et non pas seulement par certaines zones de celle-ci, le passage de l'eau vers le matériau interne 3 qui a des capacités d'absorption de l'eau par capillarité.

Les deux faces principales 2₁, 2₂ au moins quasi-parallèles si ce n'est vraiment parallèles, du fourreau externe 2 prennent en sandwich le matériau absorbant interne 3; et la face 4₃ de la partie 4 inférieure formée par l'épaisseur du matériau interne 3 qui épouse et suit au moins le bord 4₁, 4₂ inférieur desdites faces 2₁, 2₂ principales du fourreau délimitant ladite face 4₃, est plane et perméable à l'eau.

Ladite face 4₃ peut être ouverte comme la face opposée dite supérieure 4₄ mais peut être également protégée par un matériau filtre 12 perméable à l'eau mais empêchant les particules du matériau interne 3 délités par l'eau absorbée de se répandre dans le container, ou par le fourreau 2 lui-même qui envelopperait le dispositif même sur cette face 4₃ si le matériau qui le compose a les mêmes caractéristiques que ce filtre 12, comme du reste déjà proposé précédemment.

Si ce n'était pas le cas, des performations peuvent être également réalisées dans au moins une face latérale et dans la face 4₃ si elle la couvre également, du fourreau 2 afin de permettre, par ces perforations, au matériau absorbant interne 3 de capter l'eau, en particulier celle sous forme de gouttelettes présentes dans la vapeur et ce à n'importe quelle hauteur dans le container si au moins une de ses faces latérales comporte de telles perforations sur toute sa surface.

Le matériau absorbant intérieur 3 peut être constitué de feuilles 3₁ de buvard accolées les unes contre les autres parallèlement aux faces principales 2₁ et 2₂.. De telles feuilles 3₁ de buvard sont connues pour absorber de l'ordre de 100 grammes d'eau pour 80 grammes de poids sec de buvard, mais d'autres matériaux de type céramique peuvent avoir des capacités similaires.

Le fourreau externe 2 peut être un papier ou carton vernis ou non, ou un matériau de type plastique.

Le matériau filtre 12 peut être une feuille de non-tissé filtrant hydrophile.

Pour des dispositifs destinés à des containers 5 de stérilisation d'équipements comprenant au moins un panier 7 intérieur recevant les équipements à stériliser tels que représentés sur la figure 1, les dimensions du dispositif suivant l'invention sont déterminées par celles de l'espace 9 disponible entre la paroi du container 5 et celle du panier 7, et son épaisseur entre ses deux faces principales 2₁, 2₂ est apte à remplir totalement la largeur d de ce dit espace 9 au moins quand il a absorbé le maximum d'eau qui lui est possible.

A titre d'exemple, un container courant pourrait être défini comme une boîte 50 cm de long, 20 cm de haut et 20 cm de large avec une largeur d de son espace 9 de 0,5 à 1 cm : un dispositif suivant invention peut alors faire 20 cm de large et 10 cm de haut pour une épaisseur inférieure à 0,5 à 1 cm d'épaisseur.

Pour des containers de forme parallélépipédique rectangle (comme celui de l'exemple de dimensions données ci-dessus), on glisse de préférence verticalement au moins deux dispositifs 1 le long de deux parois intérieures opposées dudit container, lesquels dispositifs étant de dimensions aptes à occuper la plus grande partie de la longueur desdites parois intérieures. Si cette longueur est grande par rapport à celle d'un dispositif, il peut même être possible et nécessaire de mettre deux dispositifs l'un dans le prolongement de l'autre pour chacun des deux côtés. Ainsi, quelque soit la position, qui peut être parfois inclinée du container, dans l'appareil assurant la stérilisation, on est sûr qu'au moins un bord ou un coin inférieur d'un desdits dispositifs sera placé vers le point le plus bas du container ainsi incliné et sera alors apte à absorber l'eau qui s'accumulerait également dans cette partie la plus basse du fond du container.

## Revendications

1. Procédé de réduction de l'eau sous forme liquide présente dans les containers (5) de stérilisation d'équipements lors de la phase de stérilisation de ceux-ci, lesquels containers étant destinés pour cela à être placés dans des appareils assurant la stérilisation par la vapeur d'eau, **caractérisé en ce que** on glisse verticalement, à l'intérieur et préalablement à la fermeture du container (5), entre la paroi de celui-ci et les équipements à stériliser, au moins un dispositif (1) absorbant d'eau apte à absorber l'eau par capillarité.

2. Procédé suivant la revendication 1, **caractérisé en ce que** le dispositif étant apte à absorber l'eau par capillarité depuis au moins sa partie inférieure 4, on positionne la partie inférieure (4) dudit dispositif (1) au plus près du fond (11) du container (5).

3. Procédé suivant l'une quelconque des revendications 1 ou 2, **caractérisé en ce qu'**on utilise un dispositif absorbant (1) comportant deux faces principales (2₁, 2₂) au moins quasi-parallèles et dont l'épaisseur qui les sépare est au plus égale à une cinquième de la plus grande dimension de ces faces principales, et on dispose l'une de celles-ci en regard de la paroi du container et au moins l'une de ces faces latérales étant perméable à l'eau.

4. Procédé suivant l'une quelconque des revendications 1 à 3, **caractérisé en ce qu'**on glisse plusieurs dispositifs (1) dans le container (5), de telle sorte que la capacité totale d'absorption d'eau par ces dispositifs (1) soit supérieure à la quantité totale d'eau pouvant être présente dans le container (5) pendant un cycle de stérilisation.

5. Procédé suivant l'une quelconque des revendications 1 à 4, et dans lequel les containers (5) comprennent au moins un panier intérieur (7) recevant les équipements à stériliser, **caractérisé en ce qu'**on glisse verticalement entre la paroi du container (5) et celle du panier intérieur (7) au moins un dispositif de dimensions compatibles avec l'espace (9) disponible entre les deux dites parois.

6. Procédé suivant l'une quelconque des revendications 1 à 5 et pour un container de forme parallélépipédique rectangle, **caractérisé en ce qu'**on glisse verticalement au moins deux dispositifs le long de deux parois intérieures opposées dudit container.

7. Dispositif absorbant d'eau pour containers (5) de stérilisation d'équipements, **caractérisé en ce qu'**il est apte à être utilisé suivant le procédé de l'une quelconque des revendications 1 à 6.

8. Dispositif absorbant d'eau pour container (5) de stérilisation d'équipements, destiné pour cela à être placé dans un appareil assurant la stérilisation par la vapeur d'eau, **caractérisé en ce qu'**il a des capacités d'absorption de l'eau par capillarité, le dispositif étant de forme amincie entre deux faces principales (2₁, 2₂) au moins quasi-parallèles, et disposées en position verticale.

9. Dispositif suivant l'une quelconque des revendications 7 ou 8 , **caractérisé en ce qu'**il est semi-rigide.

10. Dispositif suivant l'une quelconque des revendications 7 à 9, **caractérisé en ce qu'**il a des capacités d'absorption de l'eau par capillarité depuis au moins sa partie dite inférieure 4 quand celle-ci est positionnée pour cela vers le bas.

11. Dispositif suivant l'une quelconque des revendications 7 à 10, **caractérisé en ce qu'**il est apte à absorber en eau au moins 50% de son poids sec et **en ce que** cette absorption se réalise par capillarité en raison de moins d'une demi-heure pour 5 cm de hauteur.

12. Dispositif suivant l'une quelconque des revendications 7 à 11, **caractérisé en ce qu'**il forme un bloc de forme générale parallélépipédique, composé d'au mois deux matériaux dont l'un extérieur (2) enveloppe l'autre intérieur (3) en laissant au moins une zone perméable à l'eau, le matériau externe (2) formant un fourreau de protection et de maintien, et le matériau interne (3) ayant des capacités d'absorption de l'eau par capillarité.

13. Dispositif selon la revendication 12, **caractérisé en ce qu'**au moins une face latérale du fourreau (2) est perméable et au moins étanche aux particules du matériau interne (3).

14. Dispositif selon l'une quelconque des revendications 12 ou 13, **caractérisé en ce que** les deux faces principales (2₁, 2₂) au moins quasi-parallèles du fourreau externe (2) prenant en sandwich le matériau absorbant interne (3), la face de la partie (4) inférieure, formée par l'épaisseur du matériau interne (3) qui épouse et suit au moins le bord (4₁, 4₂) inférieure des dites faces (2₁, 2₂) principales du fourreau, délimitant ladite face (4₃), est plane, perméable à l'eau et au moins étanche aux particules du matériau interne (3).

15. Dispositif selon l'une quelconques des revendications 11 à 14, **caractérisé en ce que** le matériau absorbant intérieur (3) est constitué de feuilles (3₁) de buvard accolées les unes contre les autres.

16. Dispositif selon l'une quelconque des revendications 7 à 15 et destiné à des containers (5) de stérilisation d'équipements comprenant un panier (7) intérieur recevant les équipement à stériliser, **caractérisé en ce que** ses dimensions sont déterminées par celles de l'espace (9) disponible entre la paroi du container (5) et celle du panier (7), et son épaisseur (e) entre ses deux faces principales (2₁, 2₂) est apte à remplir totalement la largeur (d) de ce dit espace (9) au moins quand il a absorbé le maximum d'eau qu'il lui est possible.

17. Dispositif selon l'une quelconque des revendications 7 à 16, **caractérisé en ce qu'**il comporte au moins au moins une surface métallique disposée parallèlement et entre les deux dites faces principales 2₁, 2₂.
